# FASCICULE DE BREVET EUROPEEN

(11) **EP 2 024 335 B1**
(45) Date de publication et mention de la délivrance du brevet: **23.06.2010**
(21) Numéro de dépôt: 07731364.1
(22) Date de dépôt: 26.04.2007
(51) Int. Cl.: C07D 233/54, A61K 31/4164, A61P 29/00, A61P 35/00, A61P 19/00

(54) **NOUVEAUX DERIVES D'IMIDAZOLES, LEUR PREPARATION ET LEUR UTILISATION EN TANT QUE MEDICAMENT**
NEUE IMIDAZOLDERIVATE, IHRE HERSTELLUNG UND IHRE VERWENDUNG ALS MEDIZIN
NOVEL IMIDAZOLE DERIVATIVES, PREPARATION AND USER THEREOF AS MEDICINE

(30) Priorité: 27.04.2006 FR 0603783
(43) Date de publication de la demande: 18.02.2009
(73) Titulaire: IPSEN PHARMA, 92100 Boulogne-Billancourt (FR)
(72) Inventeur: LIBERATORE, Anne-Marie, 78610 Auffargis (FR); BIGG, Dennis, 91190 Gif-sur-Yvette (FR); PONS, Dominique, 75014 Paris (FR); PREVOST, Grégoire, 92160 Antony (FR)
(74) Mandataire: Bourgouin, André
(86) Numéro de dépôt international: PCT/FR2007/000706
(87) Numéro de publication internationale: WO 2007/125197

(56) Documents cités:
- WO-A2-03/075921
- WO-A2-2004/106307

## Description

La présente demande a pour objet de nouveaux dérivés d'imidazoles. L'invention concerne également des compositions pharmaceutiques contenant ces dérivés et leur utilisation pour la préparation d'un médicament.

Les dérivés d'imidazoles selon la présente invention présentent une activité anti-tumorale et en particulier une activité inhibitrice de la polymérisation de la tubuline.

Cible de plusieurs médicaments anticancéreux, la tubuline est une protéine de petite taille qui, en se polymérisant, donne les microtubules du fuseau achromatique qui permet la division cellulaire lors de la mitose. Les vinca-alcaloïdes inhibent sa polymérisation, alors que le paclitaxel et le docétaxel la stabilisent exagérément. Dans les deux cas, la mitose ne peut se dérouler normalement, ce qui entrave la prolifération cellulaire.

En raison de leur activité anti-tumorale, les composés selon l'invention peuvent être utilisés pour le traitement de tumeurs ou de cancers comprenant les cancers de l'oesophage, de l'estomac, des intestins, du rectum, de la cavité orale, du pharynx, du larynx, du poumon, du colon, du sein, du cervix uteri, du corpus endométrium, des ovaires, de la prostate, des testicules, de la vessie, des reins, du foie, du pancréas, des os, des tissus conjonctifs, de la peau, des yeux, du cerveau, les mélanomes et les cancers du système nerveux central, ainsi que le cancer de la thyroïde, la leucémie, la maladie de Hodgkin, les lymphomes autres que ceux de Hodgkin, les myélomes multiples et autres. Par ailleurs, ces composés pourraient être également utilisés à traiter certaines infections virales telles que le syndrome immunodéficitaire acquis, l'hépatite C ainsi que des maladies auto-immunes et certaines maladies dégénératives.

Les composés selon l'invention peuvent être également utilisés pour traiter des maladies comme la goutte, des maladies rhumatismales comme par exemple la fièvre Méditerranéenne familiale et toutes autres maladies inflammatoires (Ben-Chetrit E. ; Bergmann S. ; Sood R. ; Rheumatology 2005 ; 1-9).

L'invention a donc pour objet un composé de formule générale (I) 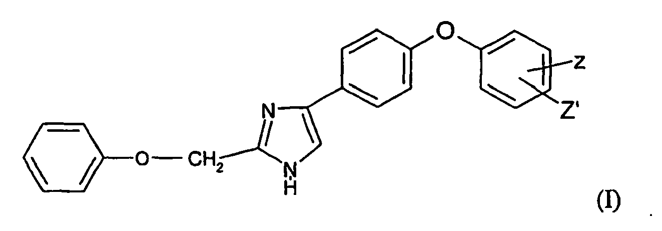 sous forme racémique, d'énantiomère ou toutes combinaisons de ces formes et dans laquelle :
Z' représente l'atome d'hydrogène ou le radical halo ;
Z représente un radical de formule -NH-X-NR₁R₂ ;
R₁ et R₂ représentent, indépendamment, l'atome d'hydrogène, un radical (C₁-C₆)alkyle, phényle éventuellement substitué par un radical (C₁-C₆)alkyle, ou benzyle éventuellement substitué sur le cycle par un radical (C₁-C₆)alkyle ;
X représente -SO₂- ou 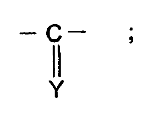
Y représente S, O, CH-R ou N-R ;
R représente -CN ou -NO₂ ;
ou un sel pharmaceutiquement acceptable de ce dernier.

L'invention couvre bien évidemment toutes les formes tautomères des composés de formule (I) tels que définis ci-dessus.

Dans les définitions indiquées ci-dessus, l'expression halo (halogéno) représente le radical fluoro, chloro, bromo ou iodo, de préférence fluoro, chloro ou bromo. L'expression (C₁-C₆)alkyle représente un radical alkyle ayant de 1 à 6 atomes de carbone, linéaire ou ramifié, tels que les radicaux méthyle, éthyle, propyle, isopropyle, butyle, isobutyle, *sec*-butyle et *tert*-butyle, pentyle ou amyle, isopentyle, néopentyle, 2,2-diméthyl-propyle, hexyle, isohexyle ou 1,2,2-triméthyl-propyle.

De préférence, l'invention concerne un composé de formule (I) tel que défini ci-dessus et caractérisé en ce que R₁ et R₂ représentent, indépendamment, l'atome d'hydrogène ou un radical (C₁-C₆)alkyle. De manière très préférentielle, R₁ représente l'atome d'hydrogène et R₂ l'atome d'hydrogène ou un radical (C₁-C₆)alkyle.

De préférence également, l'invention concerne un composé de formule (I) tel que défini ci-dessus et caractérisé en ce que X représente -C(Y)- et Y représente S, O, CH-R ou N-R. De manière très préférentielle, l'invention concerne un composé de formule (I) tel que défini ci-dessus et caractérisé en ce que X représente -C(Y)- et Y représente S, O ou N-R. De manière très préférentielle également, l'invention concerne un composé de formule (I) tel que défini ci-dessus et caractérisé en ce que X représente -C(Y)- et Y représente S ou N-R et R représente -CN.

De préférence également, l'invention concerne un composé de formule (I) tel que défini ci-dessus et caractérisé en ce que X représente -SO₂.

De préférence, l'invention concerne un composé de formules (I) tel que défini ci-dessus et caractérisé en ce que Z est en position para.

De préférence, l'invention concerne un composé de formule (I) tel que défini ci-dessus et caractérisé en ce que Z' est en position méta.

De préférence, l'invention concerne un composé de formule (I) tel que défini ci-dessus et caractérisé en ce que Z' représente le radical halo et de préférence fluoro.

L'invention a également pour objet des composés tels qu'illustrés dans la partie expérimentale et caractérisés en ce qu'ils répondent à l'une des formules suivantes :
- N-(4-méthylphényl)-N'-(4-{4-[2-(phénoxyméthyl)-1H-imidazol-4-yl]phénoxy} phényl)urée ;
- trifluoroacétate de N-butyl-N'-(4-{4-[2-(phénoxyméthyl)-1H-imidazol-4-yl]phénoxy} phényl)urée ;
- N-(4-{4-[2-(phénoxyméthyl)-1H-imidazol-4-yl]phénoxy}phényl)urée ;
- N-éthyl-N'-(4-{4-[2-(phénoxyméthyl)-1H-imidazol-4-yl]phénoxy} phényl)thiourée ;
- N-éthyl-N'-(2-fluoro-4-{4-[2-(phénoxyméthyl)-1H-imidazol-4-yl]phénoxy} phényl)thiourée ;
- N-(2-fluoro-4-{4-[2-(phénoxyméthyl)-1H-imidazol-4-yl]phénoxy}phényl)thiourée ;
- N-isopropyl-N'-(4-{4-[2-(phénoxyméthyl)-1H-imidazol-4-yl]phénoxy}phényl)urée ;
- N-(4-{4-[2-(phénoxyméthyl)-1H-imidazol-4-yl]phénoxy}phényl)-N'-phénylurée ;
- N-(4-méthylbenzyl)-N'-(4-{4-[2-(phénoxyméthyl)-1H-imidazol-4-yl]phénoxy} phényl)urée ;
- trifluoroacétate de N-éthyl-N'-(4-{4-[2-(phénoxyméthyl)-1H-imidazol-4-yl]phénoxy} phényl)urée ;
- chlorhydrate de N-(2-fluoro-4-{4-[2-(phénoxyméthyl)-1H-imidazol-4-yl]phénoxy} phényl)sulfamide ;
- N"-cyano-N-(2-fluoro-4-{4-[2-(phénoxyméthyl)-1H-imidazol-4-yl]phénoxy} phényl)guanidine ;
- N-(2-fluoro-4-{4-[2-(phénoxyméthyl)-1H-imidazol-4-yl]phénoxy}phényl)-N"-nitroguanidine ;
   et plus particulièrement à l'une des formules suivantes :

- N-(4-{4-[2-(phénoxyméthyl)-1H-imidazol-4-yl]phénoxy}phényl)urée ;
- N-éthyl-N'-(4-{4-[2-(phénoxyméthyl)-1H-imidazol-4-yl]phénoxy} phényl)thiourée ;
- N-éthyl-N'-(2-fluoro-4-{4-[2-(phénoxyméthyl)-1H-imidazol-4-yl]phénoxy} phényl)thiourée ;
- N-(2-fluoro-4-{4-[2-(phénoxyméthyl)-1H-imidazol-4-yl]phénoxy}phényl)thiourée ;
- chlorhydrate de N-(2-fluoro-4-{4-[2-(phénoxyméthyl)-1H-imidazol-4-yl]phénoxy} phényl)sulfamide ;
- N"-cyano-N-(2-fluoro-4-{4-[2-(phénoxyméthyl)-1H-imidazol-4-yl]phénoxy}phényl) guanidine ;
- N-(2-fluoro-4-{4-[2-(phénoxyméthyl)-1H-imidazol-4-yl]phénoxy}phényl)-N"-nitroguanidine.

Suivant les définitions des groupes variables Z et Z', les composés selon l'invention peuvent être préparés selon les différentes procédures A à E décrites ci-dessous.

Comme décrit dans le schéma A ci-dessous, les composés de formule générale (I) dans laquelle R₂ représente l'atome d'hydrogène et R₁ représente l'atome d'hydrogène, un radical (C₁-C₆)alkyle, phényle éventuellement substitué ou benzyle éventuellement substitué, X représente le radical -C(Y)- et Y un atome d'oxygène ou de soufre, peuvent être obtenus à partir de composés isocyanates ou isothiocyanates commerciaux de formule générale (II) selon des méthodes de synthèse organique classiques connues de l'homme du métier.

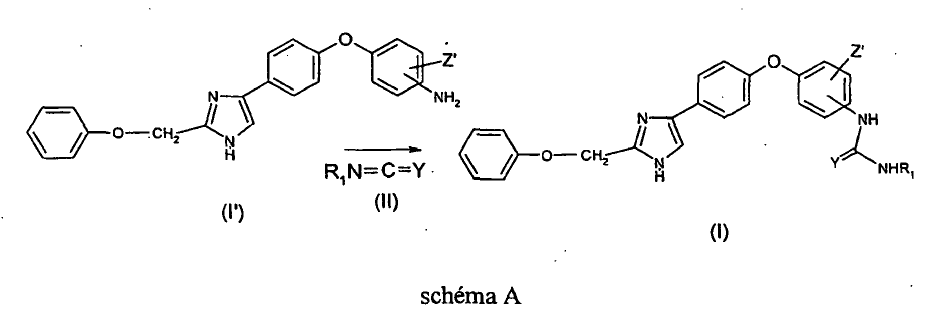

Les dérivés aniline de formule générale (I') peuvent être préparés selon la méthode décrite dans WO 2004/106307.

Comme décrit dans le schéma B ci-dessous, les composés de formule générale (I) dans laquelle R₁ et R₂ sont des atomes d'hydrogène, X représente le radical -C(Y)- et Y un atome d'oxygène ou de soufre, peuvent être, par exemple, obtenus en faisant réagir du (thio)phosgène de formule Cl₂C=Y dans un solvant inerte comme le tétrahydrofurane ou le dioxane, sur le dérivé aniline (I') pour former le dérivé iso(thio)cyanate de formule générale (III) suivi d'une condensation d'ammoniac gazeux dans un solvant tel que le tétrahydrofurane ou le dichlorométhane pour former le composé de formule générale (I).

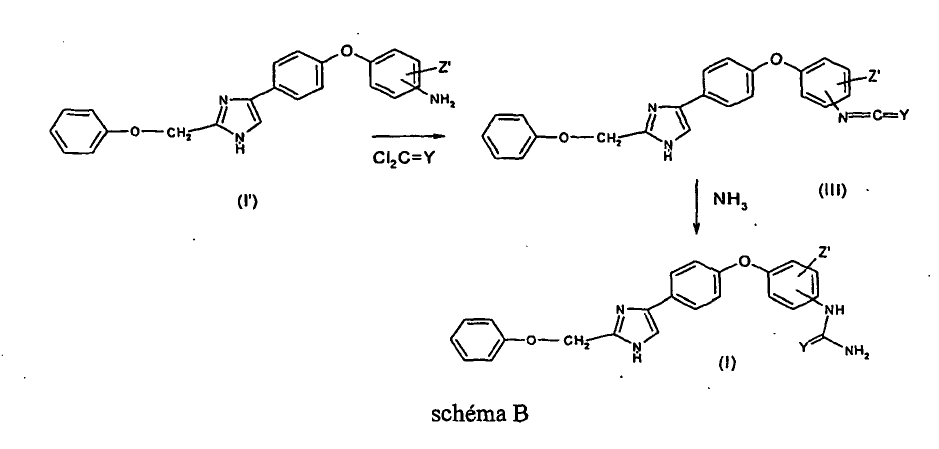

Comme décrit dans le schéma C ci-dessous, les composés de formule générale (I) dans laquelle R₁ représente l'atome d'hydrogène et R₂ l'atome d'hydrogène ou un radical alkyle, et X représente un radical -SO₂- peuvent être facilement obtenus par déprotection des intermédiaires de formule générale (V) dans laquelle Gp est un groupe protecteur pour une fonction amine (par exemple un groupe protecteur de type carbamate ou tout autre groupe protecteur connu de l'homme du métier et notamment ceux cités dans : Protective groups in organic synthesis, 2nd ed., (John Wiley & Sons Inc., 1991)). Les dérivés de formule générale (V) peuvent être obtenus en faisant réagir les composés halogénures de sulfonyle protégés par un groupement protecteur Gp tel que défini ci-dessus de formule générale (IV) que l'on prépare selon la méthode décrite dans Biorganic & Medicinal Chemistry Letters, 13 (2003), 837-840, avec le dérivé aniline de formule générale (I').

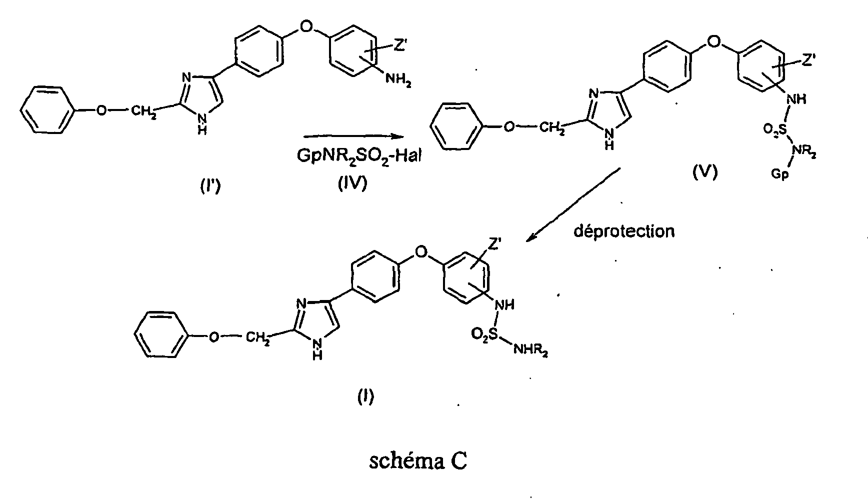

Comme décrit dans le schéma D ci-dessous, les composés de formule générale (I) dans laquelle R₁ et R₂ sont des atomes d'hydrogène, X représente le radical -C(Y)- et Y le radical N-NO₂ peuvent être facilement obtenus en chauffant au reflux d'un solvant tel que l'acétonitrile, le dérivé nitré de formule générale (VI) dans laquelle G'p est un groupe partant comme les groupements diméthylpyrazole ou pyrazole, avec le dérivé aniline de formule générale (I').

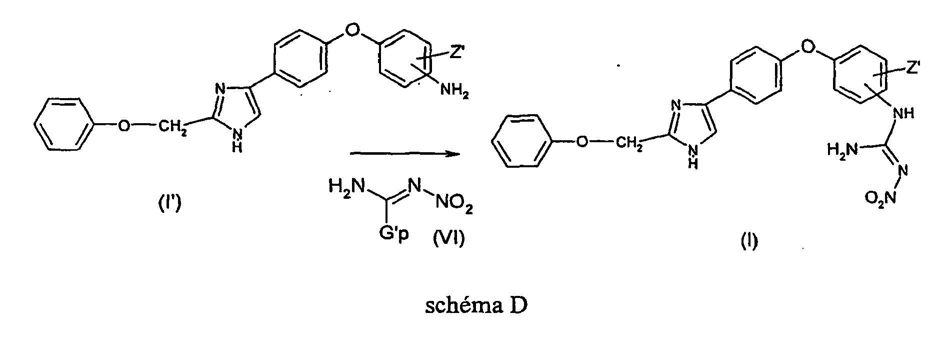

Les dérivés de formule générale (VI) peuvent être obtenus, par exemple, en faisant tout d'abord réagir dans un solvant polaire comme l'eau, la nitroguanidine et l'hydrate d'hydrazine puis en condensant le produit ainsi obtenu avec la 2,4-pentadione dans un solvant polaire comme l'eau par exemple.

Comme décrit dans le schéma E ci-dessous, les composés de formule générale (I) dans laquelle R₁ et R₂ sont des atomes d'hydrogène, X représente le radical -C(Y)- et Y le radical N-CN, peuvent être obtenus selon des méthodes connues de l'homme du métier en condensant par exemple sur le composé aniline de formule générale (I') au reflux d'un solvant tel que le diméthylformarnide, le sel de sodium du dicyanamide.

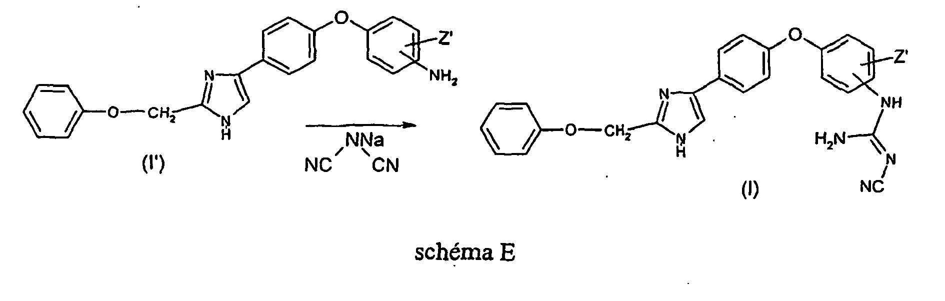

L'invention a pour objet également un procédé de préparation d'un composé de formule (I) selon l'invention et dans laquelle R₂ représente l'atome d'hydrogène, Ri représente l'atome d'hydrogène, un radical (C₁-C₆)alkyle, phényle éventuellement substitué ou benzyle éventuellement substitué, X représente le radical -C(Y)- et Y un atome d'oxygène ou de soufre, procédé caractérisé en ce que l'on fait réagir un composé de formule (I') 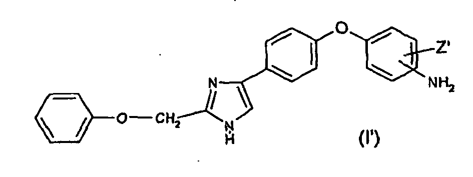 dans laquelle Z' a la signification indiquée ci-dessus, avec un composé isocyanate ou isothiocyanate de formule (II) R₁N=C=Y dans laquelle R₁ et Y ont la signification indiquée ci-dessus.

L'invention a pour objet également un procédé de préparation d'un composé de formule (I) selon l'invention et dans laquelle R₁ et R₂ sont des atomes d'hydrogène, X représente le radical -C(Y)- et Y un atome d'oxygène ou de soufre, procédé caractérisé en ce que l'on fait réagir du (thio)phosgène de formule Cl₂C=Y dans un solvant inerte sur le composé de formule (I') tel que défini ci-dessus, pour former le dérivé iso(thio)cyanate de formule générale (III) 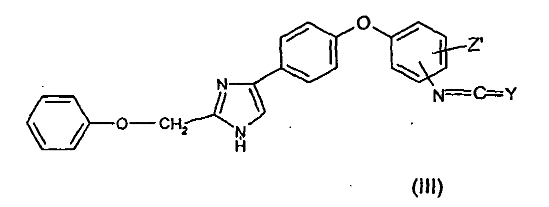 étape suivie d'une condensation d'ammoniac gazeux dans un solvant pour former le composé de formule générale (I).

L'invention a pour objet également un procédé de préparation d'un composé de formule (I) selon l'invention et dans laquelle R₁ représente l'atome d'hydrogène, R₂ représente l'atome d'hydrogène ou un radical alkyle, et X représente un radical -SO₂-, procédé caractérisé en ce que
- l'on fait réagir un composé de formule (I') tel que défini ci-dessus, avec un halogénure de sulfonyle de formule GpNR₂-SO₂-Hal dans laquelle Gp est un groupe protecteur pour la fonction amine et Hal représente un radical halo, pour former le composé (V) 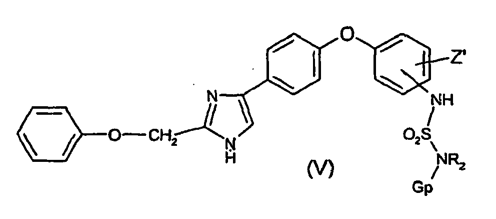 dans laquelle Z' est tel que défini à la revendication 1,
- composé (V) ainsi formé qui est déprotégé pour former le composé (I).

L'invention a pour objet également un procédé de préparation d'un composé de formule (I) selon l'invention et dans laquelle R₁ et R₂ sont des atomes d'hydrogène et X représente un radical -C(Y)- et Y le radical N-NO₂, procédé caractérisé en ce que l'on chauffe au reflux d'un solvant, un dérivé nitré de formule générale (VI) 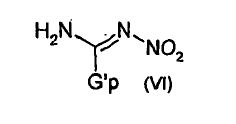 dans laquelle G'p est un groupe partant, avec le composé de formule générale (I') tel que défini ci-dessus.

L'invention a pour objet également un procédé de préparation d'un composé de formule (I) selon l'invention et dans laquelle R₁ et R₂ sont des atomes d'hydrogène et X représente un radical -C(Y)- et Y le radical N-CN, procédé caractérisé en ce que l'on condense le sel de sodium du dicyanamide avec un composé de formule (I') tel que défini ci-dessus au reflux d'un solvant.

Les composés de formule (I) selon la présente invention possèdent d'intéressantes propriétés pharmacologiques. C'est ainsi que l'on a découvert que les composés de formule (I) de la présente invention possèdent une activité anti-tumorale (anticancéreuse), et plus particulièrement une activité inhibitrice de la polymérisation de la tubuline. Ils peuvent également présenter une activité anti-rhumatismale. De plus, ils peuvent présenter une activité anti-inflammatoire.

Les composés de la présente invention peuvent ainsi être utilisés dans différentes applications thérapeutiques. Ils peuvent avantageusement être utilisés pour le traitement du cancer tels que définis précédemment et de préférence les cancers du sein, du colon, de la prostate, du pancréas et les mélanomes. Ils peuvent être également utilisés pour traiter des maladies rhumatismales ou inflammatoires comme la goutte. On trouvera ci-après, dans la partie expérimentale, une illustration des propriétés pharmacologiques des composés de l'invention.

La présente demande a également pour objet, des compositions pharmaceutiques contenant, à titre de principe actif, au moins un composé de formule (I) tel que défini ci-dessus, ainsi que les sels d'addition avec les acides minéraux ou organiques pharmaceutiquement acceptables dudit composé de formule I, en association avec un support pharmaceutiquement acceptable.

Par sel pharmaceutiquement acceptable, on entend notamment des sels d'addition d'acides inorganiques tels que chlorhydrate, bromhydrate, sulfate, phosphate, diphosphate et nitrate ou d'acides organiques tels que acétate, maléate, fumarate, tartrate, succinate, citrate, lactate, méthanesulfonate, benzènesulfonate, p-toluènesulfonate, pamoate et stéarate. Entrent également dans le champ de la présente invention, lorsqu'ils sont utilisables, les sels formés à partir de bases telles que l'hydroxyde de sodium ou de potassium. Pour d'autres exemples de sels pharmaceutiquement acceptables, on peut se référer à "Salt selection for basic drugs", Int. J. Pharm. (1986), 33, 201-217.

La présente demande a également pour objet l'utilisation d'un composé de formule (I) selon la présente invention, pour la préparation d'un médicament anti-tumoral.

La présente demande a également pour objet l'utilisation d'un composé de formule (I) selon la présente invention, pour la préparation d'un médicament destiné à inhiber la polymérisation de la tubuline.

La présente demande a également pour objet l'utilisation d'un composé de formule (I) selon la présente invention, pour la préparation d'un médicament destiné à traiter les maladies rhumatismales ou inflammatoires.

La présente demande a également pour objet l'utilisation d'un composé de formule (I) selon la présente invention, pour la préparation d'un médicament destiné à traiter la goutte.

Les composés de la présente invention peuvent être administrés seuls ou en association simultanément ou de façon séquentielle avec d'autres agents à activité anti-tumorale. Parmi les agents à activité anti-tumorale, on peut citer : les inhibiteurs de topoisomérase I tels que le diflomotécan, l'irinotécan ou le topotécan ; les inhibiteurs de topoisomèrase II ; les inhibiteurs de la polymérisation de la tubuline tels que la navelbine ; des inhibiteurs de dépolymérisation des microtubules tels que le taxol ; les agents alkylants tels que la cyclophosphamide, les fosfamides ou le melphalan ; les dérivés du platine tels que cisplatine, carboplatine ou oxaliplatine; les agents antibiotiques tels que la bléomycine ou la mitomycine ; les antimétabolites tels que le 5-fluorouracil ; les agents anti-hormonaux et les agents anti- facteurs de croissance.

L'administration d'une composition selon l'invention peut également être associée à de la radiothérapie.

La composition pharmaceutique peut être sous forme d'un solide, par exemple, des poudres, des granules, des comprimés, des gélules. Les supports solides appropriés peuvent être, par exemple, le phosphate de calcium, le stéarate de magnésium, le talc, les sucres, le lactose, la dextrine, l'amidon, la gélatine, la cellulose, la cellulose de méthyle, la cellulose carboxyméthyle de sodium, la polyvinylpyrrolidine et la cire.

Les compositions pharmaceutiques contenant un composé de l'invention peuvent aussi se présenter sous forme liquide, par exemple, des solutions, des émulsions, des suspensions ou des sirops. Les supports liquides appropriés peuvent être, par exemple, l'eau, les solvants organiques tels que le glycérol ou les glycols, de même que leurs mélanges, dans des proportions variées, dans l'eau, additionnés à des huiles ou des graisses pharmaceutiquement acceptables. Les compositions liquides stériles peuvent être utilisées pour les injections intramusculaires, intrapéritonéales ou sous-cutanées et les compositions stériles peuvent également être administrées par voie intraveineuse.

Tous les termes techniques et scientifiques utilisés dans le présent tette ont la signification connue de l'homme de l'art.

### Partie expérimentale

Suivant les définitions des groupes variables Z et Z', les composés selon l'invention peuvent être préparés selon les différentes procédures A à E décrites ci-dessus.

Les exemples sont présentés pour illustrer les procédures ci-dessus et ne doivent en aucun cas être considérés comme une limite à la portée de l'invention.

### Exemple 1 : N-(4-méthylphényl)-N'-(4-{4-[2-(phénoxyméthyl)-1H-imidazol-4-yl] phénoxy}phényl)urée

Un mélange contenant de la 4-{4-[2-(phénôxyméthyl)-1H-imidazol-4-yl] phénoxy}aniline (0,2 g, 0,56 mmol) et du paraméthylphénylisocyanate (0,089 g, 0,67 mmol) dans 3 ml de tétrahydrofurane est agité pendant 24 heures à température ambiante. Le précipité formé est filtré puis le solide est agité dans un mélange de solvants tel que éther isopropylique/acétate d'éthyle/isopropanol (5/4/1). Après filtration puis séchage, on obtient un solide sous forme d'une poudre beige avec un rendement de 73%.
RMN-¹H (δ ppm, DMSO) : 2,24 (s, 3H) ; 5,07 (s, 2H) ; 6,94-7,94 (m, 18H) ; 8,51 (s, 1H) ; 8,60 (s, 1H) ; 12,4 (sé, 1H)
MH+ expérimental = 491,20 ; M théorique = 491,56 ; Point de fusion : 192-194° C

### Exemple 2 : trifluoroacétate de N-butyl-N'-(4-{4-[2-(phénoxyméthyl)-1H-imidazol-4-yl]phénoxy}phényl)urée

Un mélange contenant de la 4-{4-[2-(phénoxyméthyl)-1H-imidazol-4-yl]phénoxy}aniline (0,2 g, 0,56 mmol) et du butylisocyanate (0,094 g, 0,84 mmol) dans 3 ml de tétrahydrofurane est agité pendant un jour à température ambiante. La résine aminométhylpolystyrène EHL (Novabiochem, 200-400 mesh, 2 % DVB) (200 mg, environ 1 mmol) est rajoufée et on laisse agiter pendant trois heures. Après filtration sur fritté puis concentration du filtrat au rotavapor, le résidu obtenu est passé sur colonne de silice RP18 (éluant : acétonitrile-acide trifluoroacétique 0,1N : 5-5) ; on obtient une poudre de couleur beige avec un rendement de 43 %.
RMN-¹H (δ ppm, DMSO) : 0,83-0,91 (m, 5H) ; 1,39-1,43 (m, 2H) ; 3,07-3,08 (m, 2H) ; 5,3 (s, 2H) ; 6,11 (m, 1H) ; 6,96-7,44 (m, 13H) ; 7,91 (m, 1H) ; 8,44 (m, 1H)
MH+ expérimental = 457,30 ; M théorique = 456,54 ; Point de fusion : 85-87° C

### Exemple 3 : N-(4-{4-[2-(phénoxyméthyl)-1H-imidazol-4-yl]phénoxy}phényl)urée

Un mélange contenant de la 4-{4-[2-(phénoxyméthyl)-1H-imidazol-4-yl]phénoxy}aniline (0,2 g, 0,56 mmol) et du triméthylsilyl-isocyanate (0,11 ml, 0,84 mmol) dans 3 ml de tétrahydrofurane est agité pendant 24 heures à température ambiante. On rajoute de la résine aminométhylpolystyrène EHL (Novabiochem, 200-400 mesh, 2 % DVB) (200 mg, environ 1 mmol) et laisse agiter pendant trois heures. Après filtration sur fritté, le filtrat est concentré au rotavapor. Au résidu obtenu on rajoute de l'acétate d'isopropyle et des traces d'isopropanol. Le mélange est chauffé jusqu'à dissolution. On filtre sur fritté puis on laisse cristalliser pendant 24 heures. Après filtration puis séchage sous cloche, on obtient une poudre de couleur beige clair.
RMN-¹H (δ ppm, DMSO) : 5,06 (s, 2H) ; 5,79 (s, 2H) ;. 6,90-7,74 (m, 14H) ; 8,50 (s, 1H) ; 12,35 (sé, 1H)
MH+ expérimental = 401,20 ; M théorique = 400,44 ; Point de fusion : 200-202° C

### Exemple 4 : N-éthyl-N'-(4-{4-[2-(phénoxyméthyl)-1H-imidazol-4-yl]phénoxy} phényl)thiourée

Un mélange contenant de la 4-{4-[2-(phénoxyméthyl)-1H-imidazol-4-yl] phénoxy}aniline (0,5 g, 1,40 mmol) et de l' éthylisothiocyanate (0,162 g, 1,70 mmol) dans 10 ml d'éthanol est chauffé pendant quatre heures au reflux. Au résidu obtenu on rajoute 5 ml de dichlorométhane et des traces d'acétate d'éthyle et d'éthanol. On agite pendant trente minutes, puis on filtre et sèche le solide obtenu. On obtient une poudre de couleur crème avec un rendement de 45 %.
RMN-¹H (δ ppm, DMSO) : 1,1 (m, 3H) ; 3,4 (m, 2H) ; 5,07 (s, 2H) ; 6,94-7,78 (m, 15H) ; 9,35 (sé, 1H) ; 12,40 (sé, 1H)
MH+ expérimental = 445,20 ; M théorique = 444,570 ; Point de fusion : 178-180° C

### Exemple 5: N-éthyl-N'-(2-fluoro-4-{4-[2-(phénoxyméthyl)-1H-imidazol-4-yl] phénoxy}phényl)thiourée

Un mélange contenant de la 2-fluoro-4-{4-[2-(pliénoxyméthyl)-1H-imidazol-4-yl] phénoxy}aniline (1 g, 2,66 mmol) et de l'éthylisothiocyanate (0,31 g, 2,32 mmol) dans 20 ml d'éthanol est chauffé pendant cinq heures au reflux. Le mélange réactionnel est concentré au rotavapor. Après passage du résidu obtenu sur colonne de silice (éluant : dichlorométhane/éthanol/ammoniaque 96/2/2), on obtient une poudre de couleur jaune avec un rendement de 11 %.
RMN-¹H (δ ppm, DMSO) : 1,06 (t, 3H) ; 3,46 (me, 2H) ; 5,08 (s, 2H) ; 6,73-7,82 (m, 15H) ; 9,03 (se, 1H)
MH+ expérimental = 463,20 ; M théorique = 462,55 ; Point de fusion : 105-107° C

### Exemple 6 : N-(2-fluoro-4-{4-[2-(phénoxyméthyl)-1H-imidazol-4-yl]phénoxy} phényl)thiourée

### 1) 4-[4-(3-fluoro-4-isothiocyanatophénoxy)phényl]-2-(phénoxyméthy1)-1H-imidazole

A 23° C et sous atmosphère d'argon, on ajoute la triéthylamine (3,34 g; 0,024 mol) à une solution contenant de la 2-fluoro-4-{4-[2-(phénoxyméthyl)-1H-imidazol-4-yl]phénoxy}aniline (3 g, 0,008 mol) dans du tétrahydrofurane (120 ml). On refroidit à 0° C puis on ajoute goutte à goutte le composé thiophosgène (1,01 g ; 0,0088 mol). On laisse agiter à cette température pendant trente minutes puis on revient à 23° C et on agite le milieu réactionnel à cette température pendant vingt minutes. On ajoute 60 ml d'eau et 150 ml d'éther éthylique au milieu réactionnel. Les deux phases sont séparées puis la phase organique est lavée avec une solution saturée en chlorure de sodium. La phase organique est séchée sur sulfate de sodium. On concentre à sec puis on triture le produit obtenu à l'isopentane avec des traces de dichlorométhane. Le solide obtenu est filtré puis séché. On obtient une poudre de couleur jaune avec un rendement de 78 %.
MH+ expérimental = 418,20 ; M théorique = 417,462

### 2) N-(2-fluoro-4-{4-[2-(phénoxyméthyl)-1H-imidazol-4-yl]phénoxy}phényl)thiourée

Le composé 4-[4-(3-fluoro-4-isothiocyanatophénoxy)phényl]-2-(phénoxyméthyl)-1H-imidazole obtenu précédemment (2,58 g, 0,0062 mol) est solubilisé à 23° C dans 50 ml d'un mélange de solvants dichIorométhane/méthanol/tétrahydrofurane 6/3/1. On refroidit à 0° C puis on fait buller de l'ammoniac gazeux jusqu'à saturation. On laisse l'agitation trente minutes à cette température puis on revient à température ambiante et laisse agiter encore trente minutes. Le milieu réactionnel est évaporé au rotavapor puis le solide obtenu est trituré dans un mélange d'éther isopropylique et d'alcool isopropylique (5:5). Le solide obtenu est filtré puis recristallisé dans un minimum d'alcool isopropylique à chaud. On laisse refroidir puis on filtre. On obtient une poudre de couleur marron clair avec un rendement de 42 %.
RMN-¹H (δ ppm, DMSO) : 5,08 (s, 2H) ; 6,79-7,82 (m, 15H) ; 9,26 (s, 1H) ; 12,4 (sé, 1H)
MH+ expérimental = 435,20 ; M théorique = 434,493 ; Point de fusion : 171-173° C

### Exempte 7 : N-isopropyl-N'-(4-{4-[2-(phénoxyméthyl)-1H-imidazol-4-yl]phénoxy} phényl)urée

Un mélange contenant de la 4-{4-[2-(phénoxymëthyl)-1H-imidazol-4-yl]phénoxy}aniline (0,2 g, 0,56 mmol) et de l'isopropylisocyanate (0,155 g, 1,8 mmol) dans 3 ml de diméthylformamide est chauffé à 100° C pendant deux heures puis agité à température ambiante pendant deux heures. On rajoute 20 ml d'eau et 30 ml d'acétate d'éthyle. On extrait puis on lave la phase organique avec une solution saturée en chlorure de sodium. La phase organique est séchée sur sulfate de sodium. Après évaporation à sec, l'huile obtenue est triturée à l'isopentane. Après filtration puis séchage, le solide obtenu est purifié sur colonne de silice (éluant dichlorométhane-éthanol-amoniaque 96/2/2) et on obtient une poudre de couleur crème rosée avec un rendement de 20 %.
RMN.¹H (δ ppm, DMSO) : 1,07 (d, 6H) ; 3,74 (m, 1H) ; 5,06 (s, 2H) ; 5,93 (m, 1H) ; 6,91-7,74 (m, 14H) ; 10,19 (s, 1H) ; 12,4 (sé, 1H)
MH+ expérimental = 443,20 ; M théorique = 442,516 ; Point de fusion : 184-186° C

### Exemple 8: N-(4-{4-[2-(phénoxyméthyl)-1H-imidazol-4-yl]phénoxy}phényl)-N'-phénylurée

Un mélange contenant de la 4-{4-[2-(phénoxyméthyl)-1H-imidazol-4-yl]phénoxy}aniline (0,2 g, 0,56 mmol) et du phénylisocyanate (0,136g, 1,12 mmol) dans 3 ml de diméthylformamide est chauffé à 100° C pendant deux heures. On revient à température ambiante puis on rajoute 20 ml d'eau et 30 ml d'acétate d'éthyle. On extrait puis on lave la phase organique avec une solution saturée en chlorure de sodium. La phase organique est séchée sur sulfate de sodium puis concentrée à sec. Le solide obtenu est purifié sur colonne de silice (éluant : dichlorométhane-éthanol-ammoniaque 96-2-2) pour obtenir une poudre de couleur blanche avec un rendement de 21 %.
RMN-¹H (δ ppm, DMSO) : 5,08 (s, 2H) ; 6,94-7,77 (m, 19H) ; 8,52 (m, 2H) ; 12,4 (sé, 1H).
MH+ expérimental = 477,20 ; M théorique = 476,534 ; Point de fusion : 146-148° C

### Exemple 9 : N-(4-méthylbenzyl)-N'-(4-{4-[2-(phénoxyméthyl)-1H-imidazol-4-yl]phénoxy}phényl)urée

Un mélange contenant de la 4-{4-[2-(phénoxyméthyl)-1H-imidazol-4-yl] phénoxy}aniline (0,2 g, 0,56 mmol) et du 4-méthylbenzylisocyanate (0,17 g, 1,12 mmol) dans 3 ml de diméthylformamide est chauffé à 100° C pendant deux heures.
On revient à température ambiante puis on rajoute 20 ml d'eau et 30 ml d'acétate d'éthyle. On extrait puis lave la phase organique avec une solution saturée en chlorure de sodium. La phase organique est séchée sur sulfate de sodium puis concentrée à sec. Le solide obtenu est agité dans 5 ml d'un mélange de solvants tel que éther éthylique/acétate d'éthyle/isopropanol 6/3/1. Le solide obtenu est filtré puis séché. On obtient une poudre de couleur crème avec un rendement de 21 %.
RMN-¹H (δ ppm, DMSO) : 4,16-4,25 (m, 2H) ; 5,07 (s, 2H) ; 6,31-6,52 (m, 1H) ; 6,91-7,75 (m, 20H) ; 8,52 (m, 1H) ; 10,18 (s, 1H) 12,4 (sé, 1H)
MH+ expérimental = 477,20 ; M théorique = 476,534 ; Point de fusion : 146-148° C

### Exemple 10 : trifluoroacétate de N-éthyl-N'-(4-{4-[2-(phénoxyméthyl)-1H-imidazol-4-yl]phénoxy}phényl)urée

Un mélange contenant de la 4-{4-[2-(phénoxyméthyl)-1H-imidazol-4-yl] phénoxy}aniline (0,2 g, 0,56 mmol) et de l'éthylisocyanate (0,066 ml, 0,84 mmol) dans 3 ml de tétrahydrofurane est agité pendant un jour à température ambiante. On rajoute de la résine aminométhylpolystyrène EHL (Novabiochem, 200-400 mesh, 2 % DVB) (200 mg, environ 1 mmol) et laisse agiter pendant trois heures. On filtre sur fritté puis on concentre le filtrat au rotavapor. Après passage du résidu obtenu sur colonne de silice RP 18 (éluant acétonitrile /acide trifluoroacétique 0,1N .5:5), on obtient un solide sous forme de gomme marron avec un rendement de 16 %.
RMN-¹H (δ ppm, DMSO) : 0,96-1,03 (m, 3H) ; 3,2-4,2 (mé, 1H) ; 3,60 (m, 2H) ; 5,29 (s, 2H) ; 6,11 (sé, 1H) ; 6,95-7,89 (m, 14H) ; 8,48 (s, 1H) ; 12-15 (sé, 1H)
MH+ expérimental = 429,20 ; M théorique = 428,90

### Exemple 11 : chlorhydrate de N-(2-fluoro-4-{4-[2-(phénoxyméthyl)-1H-imidazol-4-yl]phénoxy}phényl)sulfamide

### 1) tert-butyl {[(2-fluoro-4-{4-[2-(phénoxyméthyl)-1H-imidazol-4-yl]phénoxy}phényl) amino]sulfonyl}carbamate

A 0° C, on solubilise le composé 2-fluoro-4-{4-[2-(phénoxyméthyl)-1H-imidazol-4-yl] phénoxy}aniline (1,5 g, 4 mmol) et la triéthylamine (0,63 ml, 4,52 mmol) dans 10 ml de dichlorométhane. On rajoute à cette solution à 0° C un mélange contenant le chlorure de sulfonyle isocyanate (0,623 g, 4,40 mmol) et du tert-butanol (0,325 g, 4,40 mmol) dans 10 ml de dichlorométhane. On revient à température ambiante puis on agite pendant deux heures. Après évaporation du solvant, le solide obtenu est purifié sur colonne de silice (éluant : dichlorométhane-méthanol : 95-5). On obtient après lavage dans le dichlorométhane puis filtration, une poudre de couleur jaune avec un rendement de 75 %.

### 2) chlorhydrate de N-(2-fluoro-4-{4-[2-(phénoxyméthyl)-1H-imidazol-4-yl]phénoxy} phényl)sulfamide

A température ambiante, on fait buller jusqu'à saturation de l'acide chlorhydrique gazeux à un mélange contenant le tert-butyl {[(2-fluoro-4-{4-[2-(phénoxyméthyl)-1H-imidazol-4-yl]phénoxy}phényl)amino]sulfonyl}carbamate (1,66 g, 2,9 mmol) isolé précédemment dans un mélange de solvants tel que l'acétate d'éthyle/éthanol (3/1). On filtre le précipité puis on lave à l'éther isopropylique et isopropanol. Après séchage, on obtient un solide sous forme d'une poudre blanche avec un rendement de 80 %.
RMN-¹H (δ ppm, DMSO) : 5,43 (s, 2H) ; 6,87-8,09 (m, 15H) ; 9,01 (s, 1H) ; 12,4 (sé, 2H)
MH+ expérimental = 455,10 ; M théorique = 454,48 ; Point de fusion : 185-187° C

### Exemple 12 : N"-cyano-N-(2-fluoro-4-{4-[2-(phénoxyméthyl)-1H-imidazol-4-yl] phénoxy}phényl)guanidine

Dans un tube en verre scellé adapté au chauffage micro-ondes, on chauffe à 75° C dans un four à micro-ondes (Biotage, Emrys Optimiser) pendant 900 secondes, le composé 2-fluoro-4-{4-[2-(phénoxyméthyl)-1H-imidazol-4-yl]phénoxy}aniline (0,1 g, 0,27 mmol) et du dicyanamide de sodium (37 mg, 0,4 mmol) dans 2,5 ml de diméthylformamide et 0,5 ml d'acide chlorhydrique 1N. On évapore à sec. Après passage du résidu obtenu sur colonne de silice (éluant dichlorométhane/éthanol 93/7), on obtient un solide sous forme de poudre blanche avec un rendement de 31 %.
RMN-¹H (δ ppm, DMSO) : 5,07 (s, 2H) ; 6,82-7,81 (m, 15H) ; 8,72 (s, 1H) ; 12,40 (sé, 1H)
MH+ expérimentale = 443,20 ; M théorique = 442,452 ; Point de fusion: 139-141 ° C

### Exemple 13 : N-(2-fluoro-4-{4-[2-(phénoxyméthyl)-1H-imidazol-4-yl]phénoxy} phényl)-N"-nitroguanidine

### 1) N'-nitrohydrazinecarboximidamide

On ajoute, goutte à goutte, le composé hydrate d'hydrazine(1,05 ml, 21,6 mmol) dans une solution chauffée à 55° C contenant le composé nitroguanidine (2 g, 19,2 mmol) dans 25 ml d'eau. On agite pendant quinze minutes jusqu'à obtention d'un milieu réactionnel limpide jaune orangé. Après refroidissement, on rajoute 2 ml d'acide chlorhydrique à 37 %. Le milieu réactionnel est filtré puis le solide obtenu lavé à l'eau glacée. Le solide est ensuite recristallisé dans un minimum d'eau pour donner après filtration une poudre blanche avec un rendement de 35 %.Point de fusion : 187-189° C.

### 2) 3,5-diméthyl-N'-nitro-1H-pyrazole-1-carboximidamide

2,7 ml d'acide acétique et de 2,4-pentadione (0,785 mg, 7,83 mmol) sont ajoutés dans un mélange chauffé au reflux contenant le composé N'-nitrohydrazinecarboximidamide insolé précédemment (0,5 g, 4,20 mmol) dans 15 ml d'eau. On revient à température ambiante puis on filtre. Le solide obtenu est lavé avec du dichlorométhane. Oh obtient un solide sous forme de poudre blanche avec un rendement de 89 %.
MH+ expérimental = 184,10 ; M théorique = 183,17

### 3) N-(2-fluoro-4-{4-[2-(phénoxyméthyl)-1H-imidazol-4-yl]phénoxy}phényl)-N"-nitroguanidine

Dans un tube en verre scellé adapté au chauffage micro-ondes, on chauffe à 150° C dans un four à micro-ondes (Biotage, Emrys Optimiser) pendant 2700 secondes, la 2-fluoro-4-{4-[2-(phénoxyméthyl)-1H-imidazol-4-yl]phénoxy}aniline (0,6 g, 1,6 mmol) et la 3,5-diméthyl-N'-nitro-1H-pyrazole-1-carboximidamide (0,296 g, 1,6 mmol) dans 5 ml de méthanol. On évapore à sec. Après passage du résidu obtenu sur colonne de silice (éluant dichloromethane/éthanol 98/2), on obtient un solide sous forme de poudre blanche.
RMN-¹H (δ ppm, DMSO) : 5,08 (s, 2H) ; 6,86-7,83 (m, 13H) ; 8,2-9,5 (m, 3H) ; 12,40 (sé, 1H)
MH+ expérimental = 463,20 ; M théorique = 462,439 ; Point de fusion : 193-195° C

### Etude pharmacologique

L'activité anti-proliférative des composés de la présente invention est déterminée en appliquant la procédure expérimentale suivante :
Les différentes lignées cellulaires sont incubées à 37° C dans une atmosphère contenant 5 % de CO₂ (incubateurs Format Scientifique) dans du DMEM (milieu Eagle modifié par Dulbecco) à 4,5 g/l de glucose auquel on a rajouté 10 % de sérum de veau inactivé par la chaleur, 50 U/ml de pénicilline, 50 µg/ml de streptomycine et 2 mM de glutamine (Gibco).

L'inhibition de la prolifération cellulaire est mesurée par le test colorimétrique au WST (sel de tetrazolium, Boehringer Manheim, Meylan, France). Les cellules sont ensemencées dans des microplaques 96 puits (TPP) à raison de 1300 cellules par puits pour les DU145, et 1200 pour les MIA-Pa-Ca-2 dans 95 µl de milieu de culture. 24 heures après l'ensemencement, 5 µl de drogues sont rajoutés à différentes concentrations (le produit est dissous dans du DMA à 10-3M puis il est dilué dans du milieu de culture). Les concentrations finales vont de 500 nM à 0,97 nM. Après 72 heures d'incubation, on rajoute 10 µl de WST par puits et la détermination de l'absorbance s'effectue à 450 nm 2 heures après (Victor, Perkin Elmer).

Chaque expérience est effectuée deux fois et est le résultat de la mesure de l'absorbance de huit puits. Pour chaque produit, la mesure de l'IC₅₀ correspondant à la concentration du produit qui entraîne 50 % d'inhibition de la croissance cellulaire, est déterminée par un calcul de régression linéaire (déviation linéaire, déviation de la linéarité et différence entre les expériences, programme de calcul TSAR) sur la partie linéaire de la sigmoïde.

Les valeurs de IC₅₀ obtenues sont de l'ordre 20 nM ou inférieures, et pour certains des composés elles varient de 1 nM à 10 nM.

## Revendications

1. Composé de formule générale **(I)** 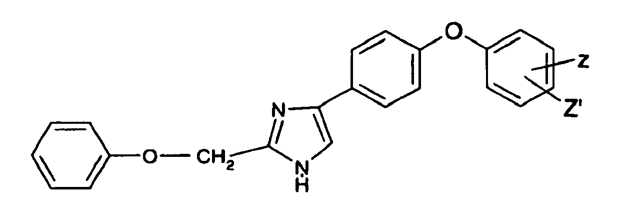 sous forme racémique, d'énantiomère ou toutes combinaisons de ces formes et dans laquelle :
Z' représente l'atome d'hydrogène ou le radical halo ;
Z représente un radical de formule -NH-X-NR₁R₂ ;
R₁ et R₂ représentent, indépendamment, l'atome d'hydrogène, un radical (C₁-C₆)alkyle; phényle éventuellement substitué par un radical (C₁-C₆)alkyle, ou benzyle éventuellement substitué sur le cycle par un radical (C₁-C₆)alkyle ;
X représente -SO₂- ou 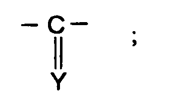
Y représente S, O, CH-R ou N-R ;
R représente -CN ou -NO₂ ; ou un sel pharmaceutiquement acceptable de ce dernier.

2. Composé selon la revendication 1, **caractérisé en ce que** R₁ et R₂ représentent, indépendamment, l'atome d'hydrogène ou un radical (C₁-C₆)alkyle.

3. Composé selon l'une des revendications précédentes, **caractérisé en ce que** R₁ représente l'atome d'hydrogène et R₂ l'atome d'hydrogène ou un radical (C₁-C₆)alkyle.

4. Composé selon l'une des revendications précédentes, **caractérisé en ce que** X représente -C(Y)- et Y représente S, O, CH-R ou N-R.

5. Composé selon l'une des revendications précédentes, **caractérisé en ce que** X représente -C(Y)- et Y représente S, O ou N-R.

6. Composé selon l'une des revendications précédentes, **caractérisé en ce que** X représente -C(Y)- et Y représente S ou N-R et R représente -CN.

7. Composé selon l'une des revendications 1 à 3, **caractérisé en ce que** X représente - SO₂.

8. Composé selon l'une des revendications précédentes, **caractérisé en ce que** Z est en position para.

9. Composé selon l'une des revendications précédentes, **caractérisé en ce que** Z' est en position méta.

10. Composé selon la revendication 9, **caractérisé en ce que** Z' représente le radical halo et de préférence fluoro.

11. Composé selon la revendication 1 **caractérisé en ce qu'**il répond à l'une des formules suivantes :
- N-(4-méthylphényl)-N'-(4-{4-[2-(phénoxyméthyl)-1H-imidazol-4-yl]phénoxy} phényl)urée ;
- trifluoroacétate de N-butyl-N'-(4-{4-[2-(phénoxyméthyl)-1H-imidazol-4-yl]phénoxy} phényl)urée ;
- N-(4-{4-[2-(phénoxyméthyl)-1H-imidazol-4-yl]phénoxy}phényl)urée ;
- N-éthyl-N'-(4-{4-[2-(phénoxyméthyl)-1H-imidazol-4-yl]phénoxy} phényl)thiourée ;
- N-éthyl-N'-(2-fluoro-4-{4-[2-(phénoxyméthyl)-1H-imidazol-4-yl]phénox} phényl)thiourée ;
- N-(2-fluoro-4-{4-[2-(phénoxyméthyl)-1H-imidazol-4-yl]phénoxy}phényl)thiourée ;
- N-isopropyl-N'-(4-{4-[2-(phénoxyméthyl)-1H-imidazol-4-yl]phénoxy}phényl)urée ;
- N-(4-{4-[2-(phénoxyméthyl)-1H-imidazol-4-yl]phénoxy}phényl)-N'-phénylurée;
- N-(4-méthylbenzyl)-N'-(4-{4-[2-(phénoxyméthyl)-1H-imidazol-4-yl]phénoxy} phényl)urée ;
- trifluoroacétate de N-éthyl-N'-(4-{4-[2-(phénoxyméthyl)-1H-imidazol-4-yl]phénoxy} phényl)urée ;
- chlorhydrate de N-(2-fluoro-4-{4-[2-(phénoxyméthyl)-1H-imidazol-4-yl]phénoxy} phényl)sulfamide ;
- N"-cyano-N-(2-fluoro-4-{4-[2-(phénoxyméthyl)-1H-imidazol-4-yl]phénoxy} phényl)guanidine ;
- N-(2-fluoro-4-{4-[2-(phénoxyméthyl)-1H-imidazol-4-yl]phénoxy}phényl)-N"-nitroguanidine.

12. Composé selon la revendication 1 **caractérisé en ce qu'**il répond à l'une des formules suivantes :
- N-(4-{4-[2-(phénoxyméthyl)-1H-imidazol-4-yl]phénoxy}phényl)urée ;
- N-éthyl-N'-(4-{4-[2-(phénoxyméthyl)-1H-imidazol-4-yl]phénoxy} phényl)thiourée ;
- N-éthyl-N'-(2-fluoro-4-{4-[2-(phénoxyméthyl)-1H-imidazol-4-yl]phénoxy} phényl)thiourée ;
- N-(2-fluoro-4-{4-[2-(phénoxyméthyl)-1H-imidazol-4-yl]phénoxy}phényl)thiourée ;
- chlorhydrate de N-(2-fluoro-4-{4-[2-(phénoxyméthyl)-1H-imidazol-4-yl]phénoxy} phényl)sulfamide ;
- N"-cyano-N-(2-fluoro-4-{4-[2-(phénoxyméthyl)-1H-imidazol-4-yl]phénoxy}phényl) guanidine ;
- N-(2-fluoro-4-{4-[2-(phénoxyméthyl)-1H-imidazol-4-yl]phénoxy} phényl)-N"-nitroguanidine.

13. Composé selon la revendication 1 **caractérisé en ce qu'**il répond à la formule suivante :
- N-(2-fluoro-4-{4-[2-(phénoxyméthyl)-1H-imidazol-4-yl]phénoxy}phényl)thiourée ;

14. Composé selon la revendication 1 **caractérisé en ce qu'**il répond à la formule suivante :
- chlorhydrate de N-(2-fluoro-4-{4-[2-(phénoxyméthyl)-1H-imidazol-4-yl]phénoxy} phényl)sulfamide.

15. Procédé de préparation d'un composé de formule (I) selon l'une des revendications précédentes et dans laquelle R₂ représente l'atome d'hydrogène, R₁ représente un atome d'hydrogène, un radical (C₁-C₆)alkyle, phényle éventuellement substitué ou benzyle éventuellement substitué, X représente le radical -C(Y)- et Y un atome d'oxygène ou de soufre, procédé **caractérisé en ce que** l'on fait réagir un composé de formule (I') 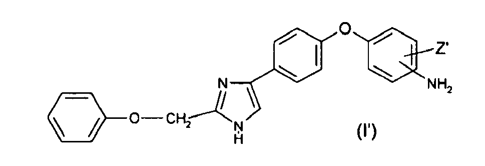 dans laquelle Z' est tel que défini à la revendication 1, avec un composé isocyanate ou isothiocyanate de formule R₁N=C=Y dans laquelle R₁ et Y ont la signification indiquée ci-dessus.

16. Procédé de préparation d'un composé de formule (I) selon l'une des revendications 1 à 14 et dans laquelle R₁ et R₂ sont des atomes d'hydrogène, X représente le radical - C(Y)- et Y un atome d'oxygène ou de soufre, procédé **caractérisé en ce que** l'on fait réagir du (thio)phosgène de formule Cl₂C=Y dans un solvant inerte sur le composé de formule (I') 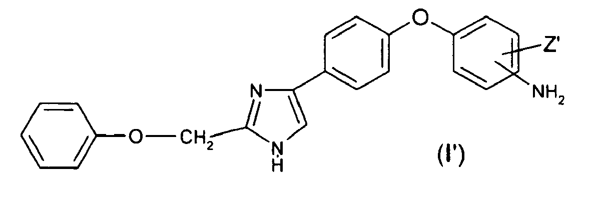 dans laquelle Z' est tel que défini à la revendication 1, pour former le dérivé iso(thio)cyanate de formule générale (III) 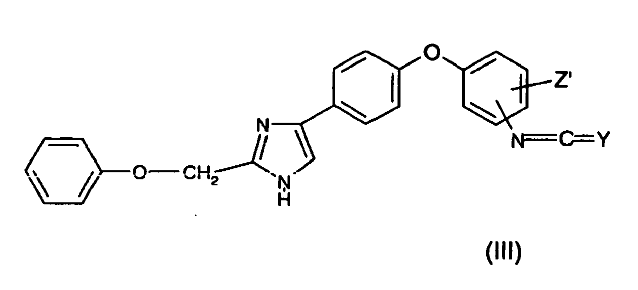 étape suivie d'une condensation d'ammoniac gazeux dans un solvant pour former le composé de formule générale (I).

17. Procédé de préparation d'un composé de formule (I) selon l'une des revendications 1 à 14 et dans laquelle R₁ représente l'atome d'hydrogène, R₂ l'atome d'hydrogène ou un radical alkyle, et X représente un radical -SO₂-, procédé **caractérisé en ce que**
- l'on fait réagir un composé de formule (I') tel que défini à la revendication 16, avec un halogénure de sulfonyle de formule GpNR₂-SO₂-Hal dans laquelle Gp est un groupe protecteur pour la fonction amine et Hal représente un radical halo, pour former le composé (V) 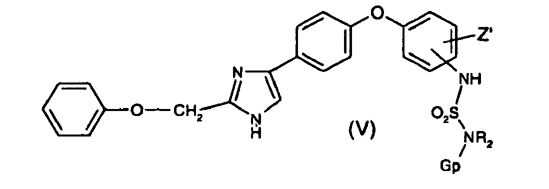
dans laquelle Z' est tel que défini à la revendication 1,
- composé (V) ainsi formé qui est déprotégé pour former le composé (I).

18. Procédé de préparation d'un composé de formule (I) selon l'une des revendications 1 à 14 et dans laquelle R₁ et R₂ sont des atomes d'hydrogène et X représente un radical - C(Y)- et Y le radical N-NO₂, procédé **caractérisé en ce que** l'on chauffe au reflux d'un solvant, un dérivé nitré de formule générale (VI) 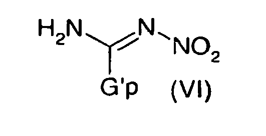 dans laquelle G'p est un groupe partant, avec le composé de formule générale (I') tel que défini à la revendication 16.

19. Procédé de préparation d'un composé de formule (I) selon l'une des revendications 1 à 14 et dans laquelle R₁ et R₂ sont des atomes d'hydrogène et X représente un radical - C(Y)- et Y le radical N-CN, procédé **caractérisé en ce que** l'on condense le sel de sodium du dicyanamide avec un composé de formule (I') tel que défini à la revendication 16, au reflux d'un solvant.

20. Composition pharmaceutique contenant, à titre de principe actif, au moins un composé selon l'une des revendications 1 à 14, en association avec un support pharmaceutiquement acceptable.

21. Utilisation d'un composé selon l'une des revendications 1 à 14, pour la préparation d'un médicament anti-tumoral.

22. Utilisation d'un composé selon l'une des revendications 1 à 14 pour la préparation d'un médicament destiné à inhiber la polymérisation de la tubuline.

23. Utilisation d'un composé selon l'une des revendications 1 à 14, pour la préparation d'un médicament destiné à traiter les maladies rhumatismales et/ou inflammatoires.

24. Utilisation d'un composé selon l'une des revendications 1 à 14, pour la préparation d'un médicament destiné à traiter la goutte.

## Claims

1. Compound of general formula **(I)** 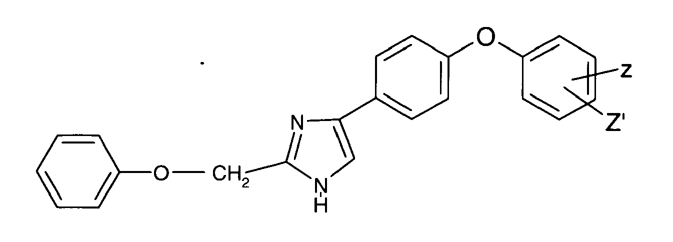 in racemic, enantiomeric form or any combination of these forms, in which:
Z' represents the hydrogen atom or the halo radical;
Z represents a radical of formula -NH-X-NR₁R₂ ;
R₁ and R₂ represent, independently, the hydrogen atom, a (C₁-C₆)alkyl radical, phenyl optionally substituted by a (C₁-C₆)alkyl radical, or benzyl optionally substituted on the ring by a (C₁-C₆)alkyl radical;
X represents -SO₂- or 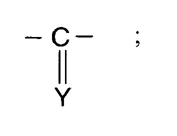
Y represents S, O, CH-R or N-R;
R represents -CN or -NO₂; or a pharmaceutically acceptable salt thereof.

2. Compound according to claim 1, **characterized in that** R₁ et R₂ represent, independently, the hydrogen atom or a (C₁-C₆)alkyl radical.

3. Compound according to one of the preceding claims, **characterized in that** R₁ represents the hydrogen atom and R₂ the hydrogen atom or a (C₁-C₆)alkyl radical.

4. Compound according to one of the preceding claims, **characterized in that** X represents -C(Y)- and Y represents S, O, CH-R or N-R.

5. Compound according to one of the preceding claims, **characterized in that** X represents -C(Y)- and Y represents S, O or N-R.

6. Compound according to one of the preceding claims, **characterized in that** X represents -C(Y)- and Y represents S or N-R and R represents -CN.

7. Compound according to one of claims 1 to 3, **characterized in that** X represents -SO₂.

8. Compound according to one of the preceding claims, **characterized in that** Z is in para position.

9. Compound according to one of the preceding claims, **characterized in that** Z' is in meta position.

10. Compound according to claim 9, **characterized in that** Z' represents the halo radical and preferably fluoro.

11. Compound according to claim 1 **characterized in that** it corresponds to one of the following formulae:
- N-(4-methylphenyl)-N'-(4-{4-[2-(phenoxymethyl)-1H-imidazol-4-yl]phenoxy} phenyl)urea;
- N-butyl-N'-(4-{4-[2-(phenoxymethyl)-1H-imidazol-4-yl]phenoxy} phenyl)urea trifluoroacetate;
- N-(4-{4-[2-(phenoxymethyl)-1H-imidazol-4-yl]phenoxy}phenyl)urea;
- N-ethyl-N'-(4-{4-[2-(phenoxymethyl)-1H-imidazol-4-yl]phenoxy} phenyl)thiourea;
- N-ethyl-N'-(2-fluoro-4-{4-[2-(phenoxymethyl)-1H-imidazol-4-yl]phenoxy} phenyl)thiourea;
- N-(2-fluoro-4-{4-[2-(phenoxymethyl)-1H-imidazol-4-yl]phenoxy}phenyl)thiourea;
- N-isopropyl-N'-(4-{4-[2-(phenoxymethyl)-1H-imidazol-4-yl]phenoxy}phenyl)urea;
- N-(4-{4-[2-(phenoxymethyl)-1H-imidazol-4-yl]phenoxy}phenyl)-N'-phenylurea;
- N-(4-methylbenzyl)-N'-(4-{4-[2-(phenoxymethyl)-1H-imidazol-4-yl]phenoxy} phenyl)urea;
- N-ethyl-N'-(4-{4-[2-(phenoxymethyl)-1H-imidazol-4-yl]phenoxy} phenyl)urea trifluoroacetate;
- N-(2-fluoro-4-{4-[2-(phenoxymethyl)-1H-imidazol-4-yl]phenoxy} phenyl)sulphamide hydrochloride;
- N"-cyano-N-(2-fluoro-4-{4-[2-(phenoxymethyl)-1H-imidazol-4-yl]phenoxy} phenyl)guanidine;
- N-(2-fluoro-4-{4-[2-(phenoxymethyl)-1H-imidazol-4-yl]phenoxy}phenyl)-N"-nitroguanidine.

12. Compound according to claim 1 **characterized in that** it corresponds to one of the following formulae:
- N-(4-{4-[2-(phenoxymethyl)-1H-imidazol-4-yl]phenoxy}phenyl)urea;
- N-ethyl-N'-(4-{4-[2-(phenoxymethyl)-1H-imidazol-4-yl]phenoxy} phenyl)thiourea;
- N-ethyl-N'-(2-fluoro-4-{4-[2-(phenoxymethyl)-1H-imidazol-4-yl]phenoxy} phenyl)thiourea;
- N-(2-fluoro-4-{4-[2-(phenoxymethyl)-1H-imidazol-4-yl]phenoxy}phenyl)thiourea;
- N-(2-fluoro-4-{4-[2-(phenoxymethyl)-1H-imidazol-4-yl]phenoxy} phenyl)sulphamide hydrochloride;
- N"-cyano-N-(2-fluoro-4-{4-[2-(phenoxymethyl)-1H-imidazol-4-yl]phenoxy}phenyl) guanidine;
- N-(2-fluoro-4-{4-[2-(phenoxymethyl)-1H-imidazol-4-yl]phenoxy}phenyl)-N"-nitroguanidine.

13. Compound according to claim 1 **characterized in that** it corresponds to the following formula:
- N-(2-fluoro-4-{4-[2-(phenoxymethyl)-1H-imidazol-4-yl]phenoxy}phenyl)thiourea.

14. Compound according to claim 1 **characterized in that** it corresponds to the following formula:
- N-(2-fluoro-4-{4-[2-(phenoxymethyl)-1H-imidazol-4-yl]phenoxy} phenyl)sulphamide hydrochloride.

15. Process for the preparation of a compound of formula (I) according to one of the preceding claims and in which R₂ represents the hydrogen atom, R₁ represents a hydrogen atom, a (C₁-C₆)alkyl, optionally substituted phenyl or optionally substituted benzyl radical, X represents the -C(Y)- radical and Y an oxygen or sulphur atom, a process **characterized in that** a compound of formula (I') 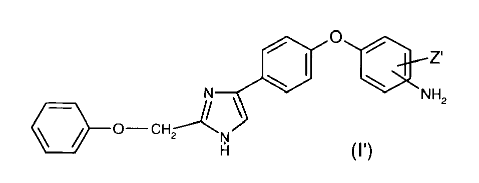 in which Z' is as defined in claim 1, is reacted with an isocyanate or isothiocyanate compound of formula R₁N=C=Y in which R₁ and Y have the meaning indicated above.

16. Process for the preparation of a compound of formula (I) according to one of claims 1 to 14 and in which R₁ and R₂ are hydrogen atoms, X represents the -C(Y)- radical and Y an oxygen or sulphur atom, a process **characterized in that** (thio)phosgene of formula Cl₂C=Y in an inert solvent is reacted with the compound of formula (I') 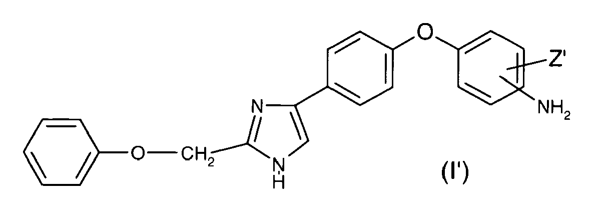 in which Z' is as defined in claim 1, in order to form the iso(thio)cyanate derivative of general formula (III) 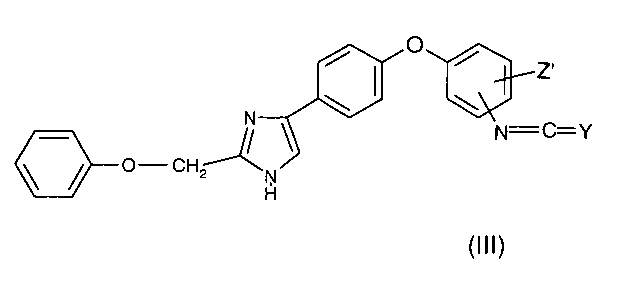 a stage followed by a condensation of gaseous ammonia in a solvent in order to form the compound of general formula (I).

17. Process for the preparation of a compound of formula (I) according to one of claims 1 to 14 and in which R₁ represents the hydrogen atom, R₂ the hydrogen atom or an alkyl radical, and X represents an -SO₂- radical, a process **characterized in that**
- a compound of formula (I') as defined in claim 14, is reacted with a sulphonyl halide of formula GpNR₂-SO₂-Hal in which Gp is a protective group for the amine function and Hal represents a halo radical, in order to form the compound (V) 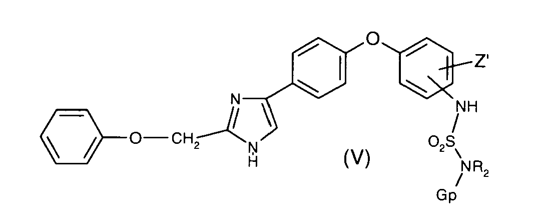
in which Z' is as defined in claim 1,
- compound (V) thus formed which is deprotected in order to form compound (I).

18. Process for the preparation of a compound of formula (I) according to one of claims 1 to 14 and in which R₁ and R₂ are hydrogen atoms and X represents a -C(Y)- radical and Y the N-NO₂ radical, a process **characterized in that** under reflux of a solvent, a nitrated derivative of general formula (VI) 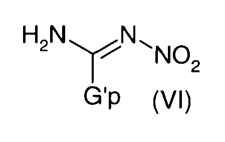 in which G'p is a leaving group, is heated with the compound of general formula (I') as defined in claim 16.

19. Process for the preparation of a compound of formula (I) according to one of claims I to 14 and in which R₁ and R₂ are hydrogen atoms and X represents a -C(Y)- radical and Y the N-CN radical, a process **characterized in that** the sodium salt of dicyanamide is condensed with a compound of formula (I') as defined in claim 16, under reflux of a solvent.

20. Pharmaceutical composition containing, as active ingredient, at least one compound according to one of claims 1 to 14, in combination with a pharmaceutically acceptable support.

21. Use of a compound according to one of claims 1 to 14, for the preparation of an anti-tumoral medicament.

22. Use of a compound according to one of claims 1 to 14, for the preparation of a medicament intended to inhibit tubulin polymerization.

23. Use of a compound according to one of claims 1 to 14, for the preparation of a medicament intended for treating rheumatic and/or inflammatory diseases.

24. Use of a compound according to one of claims 1 to 14, for the preparation of a medicament intended for treating gout.

## Patentansprüche

1. Verbindung der allgemeinen Formel (I) 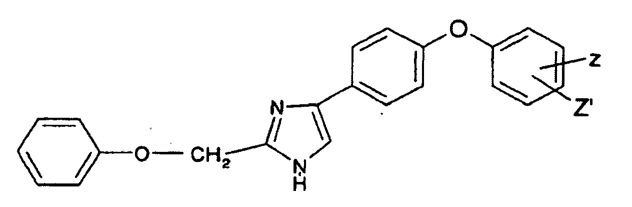 in racemischer Form, Enantiomerform oder allen Kombinationen dieser Formen, in der
Z' ein Wasserstoffatom oder einen Halogenrest darstellt;
Z einen Rest der Formel -NH-X-NR₁R₂ darstellt;
R₁ und R₂ unabhängig ein Wasserstoffatom, einen (C₁-C₆)-Alkyl-Rest; einen Phenyl-Rest, gegebenenfalls substituiert mit einem (C₁-C₆)-Alkyl-Rest, oder einen Benzyl-Rest, gegebenenfalls am Ring substituiert mit einem (C₁-C₆)-Alkyl-Rest, darstellen;
X-SO₂₋oder 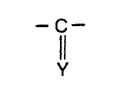 darstellt;
Y S, O, CH-R oder N-R darstellt;
R -CN oder -NO₂ darstellt;
oder ein pharmazeutisch verträgliches Salz davon.

2. Verbindung nach Anspruch 1, **dadurch gekennzeichnet, dass** R₁ und R₂ unabhängig ein Wasserstoffatom oder einen (C₁-C₆)-Alkyl-Rest darstellen.

3. Verbindung nach einem der vorangehenden Ansprüche, **dadurch gekennzeichnet, dass** R₁ ein Wasserstoffatom darstellt und R₂ ein Wasserstoffatom oder einen (C₁-C₆)-Alkyl-Rest darstellt.

4. Verbindung nach einem der vorangehenden Ansprüche, **dadurch gekennzeichnet, dass** X -C(Y)- darstellt und Y S, O, CH-R oder N-R darstellt.

5. Verbindung nach einem der vorangehenden Ansprüche, **dadurch gekennzeichnet, dass** X -C(Y)- darstellt und Y S, O oder N-R darstellt.

6. Verbindung nach einem der vorangehenden Ansprüche, **dadurch gekennzeichnet, dass** X -C(Y)- darstellt und Y S oder N-R darstellt und R -CN darstellt.

7. Verbindung nach einem der Ansprüche 1 bis 3, **dadurch gekennzeichnet, dass** X -SO₂ darstellt.

8. Verbindung nach einem der vorangehenden Ansprüche, **dadurch gekennzeichnet, dass** Z in para-Position ist.

9. Verbindung nach einem der vorangehenden Ansprüche, **dadurch gekennzeichnet, dass** Z' in meta-Position ist.

10. Verbindung nach Anspruch 9, **dadurch gekennzeichnet, dass** Z' einen Halogen-Rest und vorzugsweise einen Fluor-Rest darstellt.

11. Verbindung nach Anspruch 1, **dadurch gekennzeichnet, dass** sie einer der folgenden Formeln entspricht:
- N-(4-Methylpheny)-N'-(4-{4-[2-(phenoxymethyl)-1H-imidazol-4-yl]phenoxy}-phenyl)harnstoff;
- N-Butyl-N'-(4-(4-[2-(phenoxymethyl)-1H-imidazol-4-yl]phenoxy)phenyl)harnstoff-Trifluoracetat;
- N-(4-{4-[2-(Phenoxymethyl)-1H-imidazol-4-yl]phenoxy}phenyl)harnstoff;
- N-Ethyl-N'-(4-{4-[2-(phenoxymethyl)-1H-imidazol-4-yl]phenoxy}phenyl)thioharnstoff;
- N-Ethyl-N'-(2-fluor-4-{4-[2-(phenoxymethyl)-1H-imidazol-4-yl]phenoxy}phenyl)-thioharnstoff;
- N-(2-Fluor-4-{4-[2-(phenoxymethyl)-1H-imidazol-4-yl]phenoxy}phenyl)thioharnstoff;
- N-Isopropyl-N'-(4-{4-[2-(phenoxymethyl)-1H-imidazol-4-yl]phenoxy}phenyl)harnstoff;
- N-(4-{4-[2-(Phenoxymethyl)-1H-imidazol-4-yl]phenoxy}phenyl)-N'-phenylharnstoff;
- N-(4-Methylbenzyl)-N'-(4-{4-[2-(phenoxymethy)-1H-imidazol-4-yl]phenoxy}-phenyl)-harnstoff;
- N-Ethyl-N'-(4-{4-[2-(phenoxymethyl)-1H-imidazol-4-yl]phenoxy}phenyl)harnstoff-Trifluoracetat;
- N-(2-Fluor-4-{4-[2-(phenoxymethyl)-1H-imidazol-4-yl]phenoxy}phenyl)sulfamid-Hydrochlorid;
- N"-Cyano-N-(2-fluor-4-{4-[2-(phenoxymethyl)-1H-imidazol-4-yl]phenoxy}phenyl)-guanidin;
- N-(2-Fluor-4-{4-[2-(phenoxymethyl)-1H-imidazol-4-yl]phenoxy}phenyl)-N"-nitroguanidin.

12. Verbindung nach Anspruch 1, **dadurch gekennzeichnet, dass** sie einer der folgenden Formeln entspricht:
- N-(4-{4-[2-(Phenoxymethyl)-1H-imidiazol-4-yl]phenoxy}phenyl)harnstoff;
- N-Ethyl-N'-(4-{4-[2-(phenoxymethyl)-1H-imidazol-4-yl]phenoxy}phenyl)thioharnstoff;
- N-Ethyl-N'-(2-fluor-4-{4-[2-(phenoxymethyl)-1H-imidazol-4-yl]phenoxy}phenyl)-thioharnstoff;
- N-(2-Fluor-4-{4-[2-(phenoxymethyl)-1H-imidazol-4-yl]phenoxy}phenyl)thioharnstoff;
- N-(2-Fluor-4-{4-[2-(phenoxymethyl)-1H-imidazol-4-yl]phenoxy}phenyl)sulfamid-Hydrochlorid;
- N"-Cyano-N-(2-fluor-4-{4-[2-(phenoxymethyl)-1H-imidazol-4-yl]phenoxy}phenyl)-guanidin;
- N-(2-Fluor-4-(4-[2-(phenoxymethyl)-1H-imidazol-4-yl]phenoxy)phenyl)-N"-nitroguanidin.

13. Verbindung nach Anspruch 1, **dadurch gekennzeichnet, dass** sie der folgenden Formel entspricht:
- N-(2-Fluor-4-{4-{2-(phenoxymethyl)-1H-imidazol-4yl]phenoxy}phenyl)thioharnstoff.

14. Verbindung nach Anspruch 1, **dadurch gekennzeichnet, dass** sie der folgenden Formel entspricht:
- N-(2-Fluor-4-{4-[2-(phenoxymethyl)-1H-imidazol-4-yl]phenoxy}phenyl)sulfamid-Hydrochlorid.

15. Verfahren zur Herstellung einer Verbindung der Formel (I) nach einem der vorangehenden Ansprüche, in der R₂ ein Wasserstoffatom darstellt, R₁ ein Wasserstoffatom, einen (C₁-C₆)-Alkyl-Rest, einen gegebenenfalls substituierten Phenyl-Rest oder einen gegebenenfalls substituierten Benzyl-Rest darstellt, X den Rest -C(Y)- darstellt und Y ein Sauerstoff- oder Schwefelatom darstellt, wobei das Verfahren **dadurch gekennzeichnet ist, dass** man eine Verbindung der Formel (I') 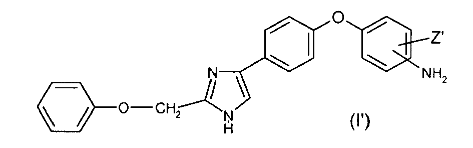 in der Z' wie in Anspruch 1 definiert ist, mit einer Isocyanat- oder Isothiocyanat-Verbindung der Formel R₁N=C=Y, in der R₁ und Y die oben angegebene Bedeutung haben, reagieren lässt.

16. Verfahren zur Herstellung einer Verbindung der Formel (I) nach einem der Ansprüche 1 bis 14, in der R₁ und R₂ Wasserstoffatome sind, X den Rest -C(Y)- darstellt und Y ein Sauerstoff- oder Schwefelatom darstellt, wobei das Verfahren **dadurch gekennzeichnet ist, dass** man (Thio)Phosgen der Formel Cl₂C=Y in einem inerten Lösungsmittel mit einer Verbindung der Formel (I') 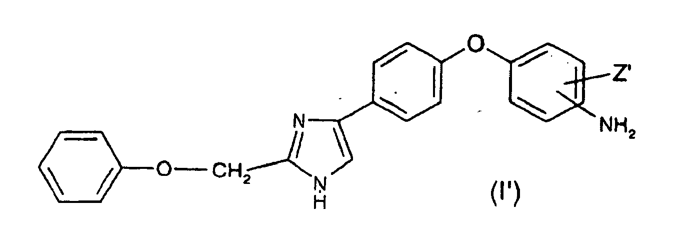 in der Z' wie in Anspruch 1 definiert ist, reagieren lässt, um das Iso(thio)cyanat-Derivat der allgemeinen Formel (III) 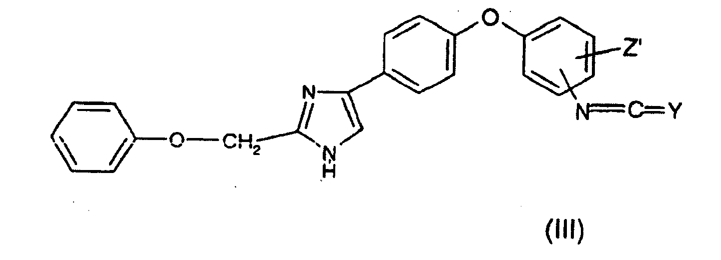 zu bilden, worauf auf diese Stufe eine Kondensation mit gasförmigem Ammoniak in einem Lösungsmittel folgt, um die Verbindung der allgemeinen Formel (I) zu bilden.

17. Verfahren zur Herstellung einer Verbindung der Formel (I) gemäß einem der Ansprüche 1 bis 14, in der R₁ ein Wasserstoffatom darstellt, R₂ ein Wasserstoffatom oder einen Alkyl-Rest darstellt und X einen Rest -SO₂- darstellt, wobei das Verfahren **dadurch gekennzeichnet ist, dass**
- man eine Verbindung der Formel (I'), wie sie in Anspruch 16 definiert ist, mit einem Sulfonylchlorid der Formel GpNR₂-SO₂-Hal, in der Gp eine Schutzgruppe für die Amin-Funktion ist und Hal einen Halogen-Rest darstellt, reagieren lässt, um die Verbindung (V) zu bilden 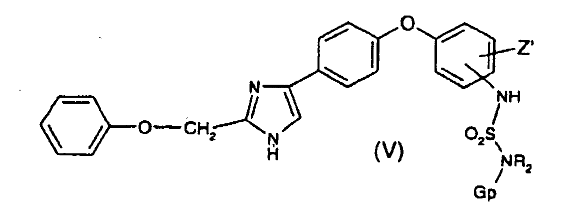 in der Z' wie in Anspruch 1 definiert ist,
- die so gebildete Verbindung (V) entschützt wird, um die Verbindung (I) zu bilden.

18. Verfahren zur Herstellung einer Verbindung der Formel (I) nach einem der Ansprüche 1 bis 14, in der R₁ und R₂ Wasserstoffatome sind und X einen Rest -C(Y)- darstellt und Y den Rest N-NO₂ darstellt, wobei das Verfahren **dadurch gekennzeichnet ist, dass** man ein nitriertes Derivat der allgemeinen Formel (VI) 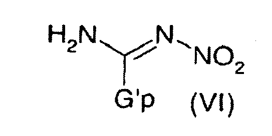 in der G'p eine Abgangsgruppe ist, mit der Verbindung der allgemeinen Formel (I'), wie sie in Anspruch 16 definiert ist, bei Rückfluss eines Lösungsmittels erwärmt.

19. Verfahren zur Herstellung einer Verbindung der Formel (I) nach einem der Ansprüche 1 bis 14, in der R1 und R2 Wasserstoffatome sind und X einen Rest -C(Y)- darstellt und Y den Rest N-CN darstellt, wobei das Verfahren **dadurch gekennzeichnet ist, dass** man das Natriumsalz von Dicyanamid mit einer Verbindung der Formel (I'), wie sie in Anspruch 16 definiert ist, kondensiert.

20. Pharmazeutische Zusammensetzung, die als Wirkstoff mindestens eine Verbindung nach einem der Ansprüche 1 bis 14 in Verbindung mit einem pharmazeutisch verträglichen Träger enthält.

21. Verwendung einer Verbindung nach einem der Ansprüche 1 bis 14 für die Herstellung eines anti-tumoralen Medikaments.

22. Verwendung einer Verbindung nach einem der Ansprüche 1 bis 14 für die Herstellung eines Medikaments, das dazu bestimmt ist, die Polymerisation von Tubulin zu inhibieren.

23. Verwendung einer Verbindung nach einem der Ansprüche 1 bis 14 für die Herstellung eines Medikaments, das dazu bestimmt ist, rheumatische und/oder inflammatorische Erkrankungen zu behandeln.

24. Verwendung einer Verbindung nach einem der Ansprüche 1 bis 14 für die Herstellung eines Medikaments, das dazu bestimmt ist, Gicht zu behandeln.
